(19) Europäisches Patentamt
European Patent Office
Office européen des brevets

(11) **EP 1 753 356 B1**

(12) **EUROPÄISCHE PATENTSCHRIFT**

(45) Veröffentlichungstag und Bekanntmachung des Hinweises auf die Patenterteilung:
**15.07.2009 Patentblatt 2009/29**

(21) Anmeldenummer: **05746317.6**

(22) Anmeldetag: **20.05.2005**

(51) Int Cl.:
*A61B 18/12* *(2006.01)*     *A61B 18/16* *(2006.01)*

(86) Internationale Anmeldenummer:
**PCT/EP2005/005512**

(87) Internationale Veröffentlichungsnummer:
**WO 2005/115262 (08.12.2005 Gazette 2005/49)**

(54) **Verfahren und Messvorrichtung zur Bestimmung der Übergangsimpendanz zwischen zwei Teilelektroden einer geteilten Neutralelektrode**

Method and measuring device for determining the transient impedance between two partial electrodes of a divided neutral electrode

Procédé et dispositif de mesure permettant de determiner l'impedance transitoire entre deux éléctrodes partielles d'une éléctrode neutre divisée

(84) Benannte Vertragsstaaten:
**DE FR GB IT**

(30) Priorität: **25.05.2004 DE 102004025613**

(43) Veröffentlichungstag der Anmeldung:
**21.02.2007 Patentblatt 2007/08**

(73) Patentinhaber: **Erbe Elektromedizin GmbH 72072 Tübingen (DE)**

(72) Erfinder: **EISELE, Florian 72074 Tübingen (DE)**

(74) Vertreter: **Bohnenberger, Johannes Meissner, Bolte & Partner GbR Postfach 86 06 24 81633 München (DE)**

(56) Entgegenhaltungen:
WO-A-96/19152          WO-A-20/04028385
US-A- 4 200 104

**Beschreibung**

Die vorliegende Erfindung betrifft ein Verfahren zur Bestimmung der Übergangsimpedanz zwischen zwei Teilelektroden einer geteilten Neutralelektrode in der Hochfrequenz-Chirurgie. Ferner wird eine Messvorrichtung zur Bestimmung der Übergangsimpedanz zwischen zwei Teilelektroden einer geteilten Neutralelektrode für eine Hochfrequenz-Chirurgieanwendung beschrieben, die einen Schwingkreis, in den die beiden Teilelektroden parallel zuschaltbar sind, und eine Stromversorgungseinrichtung aufweist, durch die eine Mess-Wechselspannung in den Schwingkreis einprägbar ist.

In der Hochfrequenz-Chirurgie wird hochfrequente elektrische Energie dem zu behandelnden Gewebe zugeführt. Hierbei wird generell zwischen einer monopolaren und einer bipolaren Anwendung des Hochfrequenzstroms unterschieden.

Bei der monopolaren Anwendung ist nur eine Aktivelektrode vorgesehen, an die eine hochfrequente Wechselspannung angelegt wird. Ferner ist die großflächige Anbringung einer Neutralelektrode am Körper des Patienten erforderlich, durch die der Stromkreis über das zwischen Aktivelektrode und Neutralelektrode liegende Gewebe geschlossen wird. Die Form der Aktivelektrode hängt von der jeweiligen Anwendung ab. Die Oberfläche der Aktivelektrode, über die Wechselstrom in das Gewebe geleitet wird, ist verhältnismäßig klein, so dass in der direkten Umgebung der Aktivelektrode eine hohe Stromdichte und demzufolge auch eine hohe Wärmeentwicklung entsteht.

Die Stromdichte nimmt mit dem Abstand von der Aktivelektrode stark ab, sofern nicht durch erhebliche Unterschiede in der Gewebeleitfähigkeit auch an weiteren Körperstellen hohe Stromdichten auftreten. Die an die Aktivelektrode angelegten Wechselströme werden über die Neutralelektrode abgeleitet. Demnach ist darauf zu achten, dass die Neutralelektrode großflächig an dem Körper des Patienten anliegt und dem hochfrequenten Wechselstrom nur einen geringen Übergangswiderstand entgegensetzt.

Bei der bipolaren Anwendung sind zwei Aktivelektroden vorgesehen, zwischen denen das zu behandelnde Gewebe aufgenommen wird. Der Stromkreis wird über das zwischen den beiden Aktivelektroden liegende Gewebe geschlossen, so dass dieses bei Anlegen einer hochfrequenten Wechselspannung erhitzt wird. Dabei fließt der Großteil des Stromes zwischen den beiden Aktivelektroden, durch Aberration kann jedoch auch ein Stromfluss in benachbarte Körperteile des Patienten auftreten. Um solche abgelenkten Ströme vom Körper großflächig abzuleiten, so dass durch sie keine unerwünschten Verbrennungen entstehen, wird zum Teil auch bei bipolaren Anwendungen eine Neutralelektrode eingesetzt, die großflächig an den Körper des Patienten anzubringen ist. Durch die Neutralelektrode wird eine erhöhte Stromdichte in anderen Körperteilen als zwischen den Aktivelektroden verhindert und unerwünschte Verbrennungen vermieden.

Tritt ein teilweises Ablösen der Neutralelektrode auf, so ist der Stromfluss auf die noch anliegenden Teile der Neutralelektrode beschränkt, was zu hohen Stromdichten und damit zu Verbrennungen am Gewebe führen kann. Wie nachfolgende Bezugnahme auf den Stand der Technik zeigt, sind unterschiedliche Überwachungssysteme bekannt, mittels derer eine Beurteilung des Übergangswiderstandes der Neutralelektrode durchführbar ist.

Ein Hochfrequenzchirurgiegerät mit einer zweigeteilten Neutralelektrode und einer Schaltung zur Bestimmung des Widerstandes zwischen den beiden Teilelektroden der Neutralelektrode ist in der DE-AS 1 139 927 beschrieben. Hierfür ist ein Gleichstromhilfskreis mit einer Gleichstromquelle vorgesehen, in den die beiden Teilelektroden derart in Reihe geschaltet sind, dass die einzige Verbindung zwischen den beiden Teilelektroden durch den Körper des Patienten gebildet wird. Überschreitet der ohmsche Widerstand, der zwischen den beiden Teilelektroden gemessen wird, einen bestimmten Grenzwert, so wird der Hochfrequenzgenerator des Chirurgiegeräts ausgeschaltet und/oder eine Alarmanlage ausgelöst.

Eine Steuerschaltung, durch die der Ausgang eines Hochfrequenz-Generators in Abhängigkeit von dem gemessenen Hochfrequenz-Strom, der zwischen einer aktiven Elektrode und einer Neutralelektrode fließt, gesteuert wird, ist aus der US 3 913 583 bekannt. Die Hochfrequenz-Stromstärke hängt von dem Scheinwiderstand zwischen aktiver Elektrode und Neutralelektrode ab, so dass die Regelung des Ausgangs des Hochfrequenz-Generators letztlich in Abhängigkeit von dem Scheinwiderstand erfolgt.

Ferner sind aus US 3 933 157, US 5 087 257 und WO 9619152 Neutralelektroden-Überwachungssysteme bekannt, mittels welcher die Anlage einer zweigeteilten Neutralelektrode an einem Patienten durch Messen des Scheinwiderstandes zwischen den beiden Teilelektroden der Neutralelektrode beurteilt wird. Hierfür ist ein Messkreis vorgesehen, in den die beiden Teilelektroden derart geschaltet sind, dass der Messkreis über das zwischen den beiden Teilelektroden liegende Gewebe des Patienten geschlossen wird. Zur Bestimmung des Scheinwiderstandes wird eine Wechselspannung an den Messkreis angelegt.

Aus US 4 200 104 ist ein Überwachungssystem für eine zweigeteilte Neutralelektrode bekannt, das über Kapazitätsmessungen zwischen den beiden Teilelektroden der Neutralelektrode ein partielles Ablösen der Neutralelektrode vom Patienten feststellen soll. Die vorgeschlagene Schaltung zur Bestimmung der Kapazität weist einen monostabilen Multivibrator auf, dem ein Signal konstanter Frequenz als Eingangssignal zugeführt wird. Die beiden Teilelektroden der Neutralelektrode sind derart in die Multivibrator-Schaltung eingebaut, dass die Kapazität zwischen diesen beiden Teilelektroden die Pulsweite des Ausgangssignals des monostabilen Multivibrators beeinflusst. Durch Auswerten der Pulsweite soll die Kapazität ermittelt und anhand dieser auf die Anlagefläche der Neutralelektrode an den Patienten geschlossen werden. Die Pulsweite der Multivibrator-Schaltung wird jedoch auch durch den ohmschen Widerstand zwischen den beiden Teilelektroden beeinflusst, der in Abhängigkeit von der Anlagefläche der Neutralelektrode ebenfalls Änderungen unterliegt. Die Trennung der Einflüsse, die durch die Kapazität bedingt sind, von denen, die durch den ohmschen Widerstand bedingt sind, scheint bei der vorgeschlagenen Schaltung nicht möglich zu sein.

DE 197 14 972 A1 beschreibt eine Einrichtung zur Überwachung der Applikation einer zweigeteilten Neutralelektrode, wobei die Einrichtung einen Impedanzsensor, der die in Serie geschalteten Übergangswiderstände der beiden Teilelektrodenflächen erfasst, aufweist. Die Einrichtung ist mittels eines Transformators von den Teilelektrodenflächen galvanisch getrennt. Bei der Ermittlung des Patientenübergangswiderstandes wird ausgenutzt, dass dieser Widerstand jeweils parallel zu dem Transformator und zu einer Kapazität angeordnet ist, und demnach ein Parallel-Schwingkreis gebildet wird. Durch Anregung des gedämpften Schwingkreises mit seiner Resonanzfrequenz wird die Spannung alleine durch den Patientenübergangswiderstand bestimmt. Demgemäß lässt die bei der Resonanzfrequenz gemessene Spannung Rückschlüsse auf den Patientenübergangswiderstaad zu.

Der Übergang zwischen dem Körper des Patienten und der Neutralelektrode setzt dem hochfrequenten Wechselstrom neben einem ohmschen Widerstand auch einen durch Aufladungseffekte bedingten kapazitiven Widerstand entgegen. Die genannten Überwachungssysteme des Standes der Technik beschränken sich darauf, entweder den ohmschen Widerstand oder den Scheinwiderstand dieser Übergangsimpedanz zu bestimmen.

Ferner beschreibt US 4 200 104 eine Vorrichtung zum Messen der Kapazität und damit zum Abschätzen der Anlagefläche der Neutralelektrode, jedoch wird die vorgeschlagene Kapazitätsmessung der Messvorrichtung durch den ohmschen Anteil der Übergangsimpedanz beeinflusst.

Die WO 2004/028 385 A1 beschreibt ein System zur Erfassung der Impedanz zwischen einem Patienten und einer Rückelektrode (return pad) in einer Elektrochirurgie-Vorrichtung.

Der vorliegenden Erfindung liegt die Aufgabe zugrunde, eine Messvorrichtung und ein Verfahren bereitzustellen, welche/welches eine bessere Beurteilung der Anlage der Neutralelektrode ermöglicht.

Diese Aufgabenstellung wird durch ein erfindungsgemäßes Verfahren nach Anspruch 1 und durch eine Messvorrichtung nach Anspruch 10 gelöst. Vorteilhafte Ausführungsformen sowie ein Hochfrequenz-Chirurgiegerät sind den weiteren Ansprüchen zu entnehmen.

Bei der vorliegenden Erfindung wird die Tatsache berücksichtigt, dass die Übergangsimpedanz aus einem ohmschen Widerstand und aus einem kapazitiven Widerstand gebildet wird. Die Übergangsimpedanz wird in Vektordarstellung als Vektorsumme eines reellen Vektors für den ohmschen Widerstand und eines komplexen Vektors für den kapazitiven Widerstand dargestellt, wobei der Betrag der Übergangsimpedanz als Scheinwiderstand bezeichnet wird.

Da der kapazitive Widerstand frequenzabhängig ist, liefert die alleinige Messung des Scheinwiderstandes bei einer Testfrequenz keine verlässliche Aussage über die Größe des Scheinwiderstandes bei Hochfrequenz-Anwendungen. Dazu müsste der relative Anteil des kapazitiven Widerstandes an dem Scheinwiderstand bekannt sein. Demgemäß liegt ein wesentlicher Gedanke der Erfindung darin, ein Verfahren und eine Messvorrichtung bereitzustellen, durch welches/welche der ohmsche Anteil bzw. der kapazitive Anteil der Übergangsimpedanz separat messbar ist. Insbesondere die Bestimmung des rein kapazitiven Anteils der Übergangsimpedanz ermöglicht Aussagen über die Größe der Fläche, mit der die Neutralelektrode an dem Körper des Patienten anliegt. Ferner kann aus dem erhaltenen kapazitiven Widerstand bei einer Testfrequenz auch der kapazitive Widerstand bei einer Hochfrequenz-Anwendung berechnet werden, wodurch der Chirurg weitere wertvolle Informationen erhält, die ihm eine sinnvolle Einstellung der Stromamplitude und der Frequenz für die Hochfrequenz-Anwendung ermöglichen.

Vorteilhaft für die Bestimmung der Übergangsimpedanz ist die Verwendung einer geteilten Neutralelektrode, insbesondere einer zweigeteilten Neutralelektrode, deren beide Teilelektroden elektrisch voneinander isoliert sind und die beide an das Gewebe, insbesondere an die Haut, des Patienten angelegt werden. Die beiden Teilelektroden können derart in einen Messkreis eingebaut werden, dass eine elektrische Verbindung zwischen den beiden Elektroden nur durch das Gewebe des Patienten vorliegt. Die Übergangsimpedanz zwischen den beiden Elektroden wird durch die beiden Teilimpedanzen an dem Übergang, zwischen der jeweiligen Teilelektrode und dem Gewebe des Patienten, und durch die Leitfähigkeit des dazwischenliegenden Gewebes bestimmt. Demnach wird die Übergangsimpedanz zwischen den beiden Teilelektroden allgemein als Maß für die Anlage der Neutralelektrode auf dem Gewebe des Patienten herangezogen.

Ein erfindungsgemäßes Verfahren ist demgemäß zur Bestimmung der Übergangsimpedanz zwischen zwei Teilelektroden einer geteilten Neutralelektrode in der Hochfrequenz-chirurgie ausgelegt und zeichnet sich dadurch aus, dass mit dem Verfahren neben dem ohmschen Anteil der rein kapazitive Anteil der Übergangsimpedanz bestimmt wird.

Zum Bestimmen des kapazitiven Anteils der Übergangsimpedanz wird ein Schwingkreis bzw. Parallelschwingkreis eingesetzt, mit einer parallel zu einer Kapazität geschalteten Induktivität. Dabei wird ausgenutzt, dass die Resonanzbedingung des Schwingkreises von der darin vorgesehenen Kapazität, Induktivität und dem ohmschen Widerstand, falls ein solcher vorgesehen ist, abhängt. Die Resonanzfrequenz verändert sich, wenn parallel zu dem Schwingkreis die beiden Teilelektroden zugeschaltet werden, so dass die Übergangsimpedanz parallel zu dem Schwingkreis geschaltet ist. Mit dem erfindungsgemäßen Verfahren wird ein Bestimmen des kapazitiven Anteils der Übergangsimpedanz durchgeführt, indem die Resonanzfrequenz-Verschiebung gemessen wird, die durch ein paralleles Zuschalten der beiden Teilelektroden in den Schwingkreis bedingt ist.

Das Bestimmen des rein kapazitiven Anteils der Übergangsimpedanz wird vorzugsweise gemäß der nachfolgend beschriebenen Schritte durchgeführt. Für die erste Messung ist ein Schwingkreis mit nicht zugeschalteter Übergangsimpedanz bereitzustellen, der als Basisschwingkreis bezeichnet wird. Dieser weist eine Messinduktivität und eine Kopplungskapazität auf. Für die Bereitstellung der Kopplungskapazität ist vorzugsweise auf die allgemein eingesetzten Kopplungskondensatoren zurückzugreifen, die jeweils in einem Zweig einer verzweigten Neutralelektrodenzuleitung angeordnet sind, und eine gleichmäßige Stromverteilung auf die beiden Teilelektroden unterstützen sollen. Die Messinduktivität wird parallel zu der Kopplungskapazität geschaltet und dient der Vervollständigung des Schwingkreises. Vorzugsweise wird als Messinduktivität eine Spule eingesetzt, deren Induktivität ausgelegt ist, eine geeignete Resonanzfrequenz des Basisschwingkreises einzustellen. Während die Kopplungskondensatoren vorzugsweise in dem Basisschwingkreis und in dem Hochfrequenz-Stromkreis angeordnet sind, ist die Messinduktivität vorzugsweise nur in dem Basisschwingkreis und nicht in dem Hochfrequenz-Stromkreis angeordnet.

In der ersten Messung wird dann eine Resonanzfrequenz des genannten Basisschwingkreises, die im Folgenden als Basisresonanzfrequenz bezeichnet wird, durchgeführt.

Für die zweite Messung wird ein erweiterter Schwingkreis verwendet, der durch paralleles Zuschalten der Übergangsimpedanz in den Basisschwingkreis bereitgestellt wird. In der zweiten Messung wird die Resonanzfrequenz des erweiterten Schwingkreises gemessen, die im Folgenden als Probenresonanzfrequenz bezeichnet wird.

Die Ermittlung des kapazitiven Anteils der Übergangsimpedanz basiert auf einem Modell, gemäß dem die Übergangsimpedanz durch einen rein ohmschen Widerstand, der parallel zu einem rein kapazitiven Widerstand geschaltet ist, hinreichend genau darstellbar ist. Dieses Modell stellt eine gute Näherung der tatsächlichen Übergangsimpedanz dar. Basierend auf diesem Modell ist aus der Basisresonanzfrequenz, der Probenresonanzfrequenz und der Messinduktivität der kapazitive Anteil der Übergangsimpedanz ermittelbar.

Vorzugsweise wird für die Berechnung des kapazitiven Widerstandes die zugehörige Kapazität $C_x$ des

Übergangs der beiden Teilelektroden aus der gemessenen Basisresonanzfrequenz $f_1$, der gemessenen Probenresonanzfrequenz $f_2$ und der Messinduktivität L berechnet.

Für die Resonanzbedingung des Basisschwingkreises gilt nachfolgende Formel, wobei die Kopplungskapazität des Basisschwingkreises mit $C_0$ bezeichnet wird:

$$2\pi f_1 C_0 - \frac{1}{2\pi f_1 L} = 0$$

Für die Resonanzbedingung des erweiterten Schwingkreises gilt die folgende Formel:

$$2\pi f_2(C_0 + C_x) - \frac{1}{2\pi f_2 L} = 0$$

Werden die beiden Resonanzbedingungen ineinander eingesetzt, so erhält man für die Kapazität $C_x$ des Übergangs zwischen den beiden Teilelektroden:

$$C_x = \frac{1}{4\pi^2 L}\left(\frac{f_1^2 - f_2^2}{f_1^2 f_2^2}\right)$$

Der kapazitive Widerstand $X_c$ für eine bestimmte Frequenz f wird anhand folgender Formel berechnet:

$$X_c = \frac{1}{2\pi f C_x}$$

Im Resonanzfall des Basisschwingkreises bzw. des erweiterten Schwingkreises sind Strom und Spannung in einer Zuleitung zu dem jeweiligen Schwingkreis in Phase. Diese Phasenbedingung des Resonanzfalls wird vorzugsweise zur Bestimmung der Basisresonanzfrequenz und der Probenresonanzfrequenz herangezogen. Insbesondere wird vorgeschlagen, dass zum Messen der Basisresonanzfrequenz des Basisschwingkreises bzw. der Probenresonanzfrequenz des erweiterten Schwingkreises eine Mess-Wechselspannung an den Basisschwingkreis bzw. an den erweiterten Schwingkreis angelegt wird, und die Phasenlage zwischen Strom und Spannung in einer Zuleitung zu dem entsprechenden Strom gemessen wird. Die Frequenz der Mess-Wechselspannung wird angepasst, bis Strom und Spannung in Phase sind, und demgemäß der entsprechende Schwingkreis in Resonanz ist. Dabei kann die Frequenz innerhalb eines bestimmten Frequenzbereichs durchgefahren werden, bis die Resonanzbedingung festgestellt wird. Vorzugsweise ist jedoch eine Steuerung vorgesehen, die in Abhängigkeit von der gemessenen Phasenlage die Frequenz der Mess-Wechselspannung anhand eines Regelalgorithmus, insbesondere anhand eines Proportional-Integral-Regelalgorithmus, an die Resonanzfrequenz anpasst.

Weitere Vorteile ergeben sich, wenn zur Erzeugung der Mess-Wechselspannung eine Konstantstromquelle verwendet wird, die ein rechteckförmiges Wechselstromsignal konstanter Stromamplitude an den Basisschwingkreis bzw. an den erweiterten Schwingkreis ausgibt. Insbesondere ist vorteilhaft, wenn die Frequenz des Wechselstromsignals mittels eines Timersignals, das insbesondere von einer Steuerung gesteuert wird, einstellbar ist, so dass der Nulldurchgang und die Frequenz des Wechselstromsignals bereits durch das Timersignal bekannt sind.

Ferner ist bekannt, dass im Resonanzfall des Basisschwingkreises bzw. des erweiterten Schwingkreises die Impedanz des Schwingkreises allein durch den ohmschen Widerstand bestimmt wird. Im Resonanzfall ist demnach die Spannung über dem jeweiligen Schwingkreis maximal. Diese Resonanzbedingung kann zum Messen der Basisresonanzfrequenz des Basisschwingkreises bzw. zum Messen der Probenresonanzfrequenz des erweiterten Schwingkreises ausgenutzt werden. Hierfür ist an dem jeweiligen Schwingkreis eine Mess-Wechselspannung anzulegen und die Spannung über dem jeweiligen Schwingkreis zu messen. Die Frequenz der Mess-Wechselspannung wird angepasst, bis die Spannung einen Maximalwert erreicht. Vorzugsweise wird als Stromquelle eine Konstantstromquelle eingesetzt.

Wie bereits oberhalb diskutiert wurde, ist die Bereitstellung eines Verfahrens und einer Messvorrichtung wünschenswert, durch welches/welche der ohmsche Anteil bzw. der kapazitive Anteil der Übergangsimpedanz separat messbar ist. Demnach wird bei dem Verfahren vorzugsweise auch der ohmsche Anteil der Übergangsimpedanz bestimmt. Dadurch ist auch die Übergangsimpedanz sowie die relativen Anteile des ohmschen Widerstandes und des kapazitiven Widerstandes an der Übergangsimpedanz bekannt, so dass eine fundierte Beurteilung der Anlage der Neutralelektrode gelingt. Insbesondere kann der Phasenwinkel der Übergangsimpedanz anhand des ohmschen und des kapazitiven Anteils berechnet werden.

Zur Bestimmung des ohmschen Widerstandes wird die Tatsache ausgenützt, dass im Resonanzfall des Basisschwingkreises bzw. des erweiterten Schwingkreises die Übergangsimpedanz allein durch den ohmschen Widerstand gegeben ist. Wird im Resonanzfall der Strom in einer Zuleitung zu dem erweiterten Schwingkreis und die Spannung über dem erweiterten Schwingkreis gemessen, so kann daraus direkt der ohmsche Widerstand der Übergangsimpedanz berechnet werden, sofern der ohmsche Widerstand des Basisschwingkreises vernachlässigbar klein ist. Der ohmsche Widerstand ergibt sich durch Dividieren eines Spitzenwertes des Spannungssignals durch einen Spitzenwert des Stromsignals. Weist der Basisschwingkreis hingegen einen erheblichen ohmschen Widerstand auf, so ist dieser analog im Basisschwingkreis bestimmbar.

Da sich die Teilelektroden auch während einer Hochfrequenz-Anwendung ablösen können, ist empfehlenswert, zur Überwachung der Übergangsimpedanz die Probenresonanzfrequenz des erweiterten Schwingkreises in zeitlichen Abständen zu messen.

Ferner ist vorteilhaft, wenn feststellbar ist, welche der beiden Teilelektroden schlechter anliegt. Dazu wird vorzugsweise, insbesondere während einer Hochfrequenz-Chirurgieanwendung, jeweils der von einer Teilelektrode abfließende Strom gemessen.

Vorrichtungsseitig betrifft die Erfindung eine Messvorrichtung zur Bestimmung der Übergangsimpedanz zwischen zwei Teilelektroden einer geteilten Neutralelektrode für eine Hochfrequenz-Chirurgieanwendung. Die Messvorrichtung weist einen Schwingkreis, in den die beiden Teilelektroden parallel zuschaltbar sind, und eine Stromversorgungseinrichtung auf, mittels welcher eine Mess-Wechselspannung in den Schwingkreis einprägbar ist.

Die entsprechende Umsetzung des Erfindungsgedankens in eine Messvorrichtung sieht vor, dass die Messvorrichtung zum Bestimmen des rein kapazitiven Anteils der Übergangsimpedanz ausgebildet ist. Hierfür ist eine Steuerung und eine Phasenmesseinrichtung vorgesehen, wobei die Stromversorgungseinrichtung, die Steuerung und die Phasenmesseinrichtung derart ausgelegt sind, dass die Phase des Stromes und der Spannung der an den Schwingkreis angelegten Mess-Wechselspannung über die Phasenmesseinrichtung von der Steuerung erfassbar ist, und dass die Frequenz der Stromversorgungseinrichtung durch die Steuerung derart steuerbar ist, dass Strom und Spannung in Phase sind.

Kosten werden eingespart, wenn die Messvorrichtung auf Patientenkreispotential betrieben wird. Eine galvanische Trennung zwischen Teilen der Messvorrichtung und Patientenkreispotential, die in der Regel durch kostenintensive Transformatoren realisiert wird, ist dann nicht nötig. Es ist lediglich eine galvanische Trennung der Stromversorgungseinrichtung und weiterer Teile der Messvorrichtung von dem Zwischenstromkreis des Hochfrequenzchirurgiegerätes erforderlich, die beispielsweise durch DC/DC-Wandler und Optokoppler realisierbar ist. Demgemäß wird erfindungsgemäß die Mess-Wechselspannung von der Stromversorgungseinrichtung direkt in den Schwingkreis eingespeist.

Bei nicht zugeschalteten Teilelektroden entspricht der Schwingkreis dem Basisschwingkreis und weist eine Kopplungskapazität und eine dazu parallel geschaltete Messinduktivität auf. Wie bereits mit Bezug auf das erfindungsgemäße Verfahren erwähnt, werden die Koppelkondensatoren, die jeweils in einem Zweig der verzweigten Neutralelektrodenzuleitung angeordnet sind, als Kopplungskapazität verwendet.

Ferner ist vorteilhaft, wenn die Resonanzbedingung des Schwingkreises anhand der Phasenbeziehung zwischen Strom und Spannung, insbesondere in einer Zuleitung zu dem Schwingkreis, feststellbar ist. Hierzu ist die Stromversorgungseinrichtung vorzugsweise eine Konstantstromquelle, deren Frequenz mittels eines Timersignals von der Steuerung einstellbar ist und die als Ausgang einen rechteckförmigen Wechselstrom erzeugt.

Demgemäß sind von der Steuerung der Stromnulldurchgang und die angelegte Frequenz anhand des Timersignals erfassbar. Vorzugsweise ist ein Analogkomparator vorgesehen, über den der Spannungsnulldurchgang in einer Zuleitung zu dem Schwingkreis von der Steuerung feststellbar ist, so dass die Steuerung mittels des Timersignals und des Analogkomparators den Phasenunterschied zwischen Strom und Spannung erfassen kann.

Insbesondere zur Bestimmung des ohmschen Widerstandes ist vorzugsweise eine Spannungsmesseinrichtung zum Messen der Spannung über dem Schwingkreis vorgesehen, so dass ein Spitzenwert der Spannung bei Resonanzbedingung des Schwingkreises mit zugeschalteter Übergangsimpedanz messbar ist.

Insbesondere zur Beurteilung, welche der beiden Teilelektroden besser anliegt, ist an jedem Leitungszweig, der zu einer Teilelektrode der Neutralelektrode führt, ein Stromsensor angeordnet, durch den der von der jeweiligen Teilelektrode abfließende Strom messbar ist.

Weitere vorteilhafte Ausgestaltungen der vorgeschlagenen Messvorrichtung ergeben sich weitgehend aus den obigen Erläuterungen zum erfindungsgemäßen Verfahren gemäß den wesentlichen Merkmalen der Erfindung sowie deren vorteilhaften Ausgestaltungen, umgesetzt in eine schaltungstechnische Realisierung in der Messvorrichtung.

Ferner betrifft die Erfindung ein Hochfrequenz-Chirurgiegerät mit mindestens einer Aktivelektrode und einer geteilten Neutralelektrode, das eine erfindungsgemäße Messvorrichtung zur Bestimmung der Übergangsimpedanz zwischen zwei Teilelektroden der geteilten Neutralelektrode aufweist.

Vorteile und Zweckmäßigkeiten der Erfindung ergeben sich im Übrigen anhand der nachfolgenden Beschreibung eines bevorzugten Ausführungsbeispiels anhand der Zeichnung.

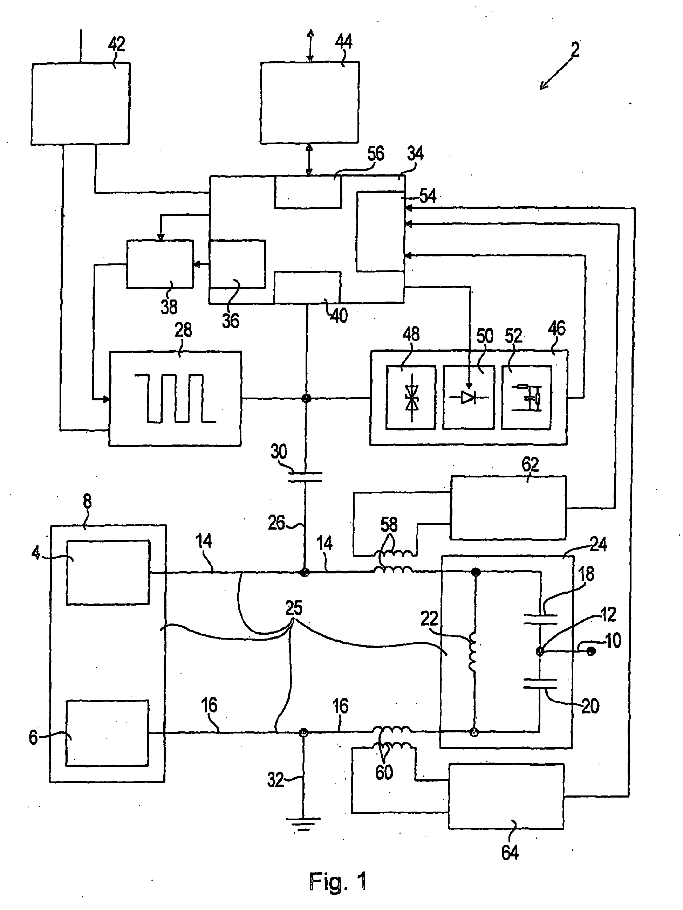

Fig. 1 zeigt die Schaltungsanordnung einer erfindungsgemäßen Messvorrichtung.

Mit der gezeigten Messvorrichtung 2 ist sowohl der kapazitive Anteil als auch der ohmsche Anteil der Übergangsimpedanz zwischen zwei Teilelektroden 4 und 6 einer Neutralelektrode 8 bestimmbar. Die beiden Teilelektroden 4, 6 sind elektrisch voneinander isoliert, wobei durch Anlegen beider Teilelektroden 4 und 6 an den Körper eines Patienten eine elektrische Verbindung zwischen den beiden Teilelektroden 4, 6 durch das Gewebe des Patienten hergestellt wird. In der Regel wird die Bestimmung der Übergangsimpedanz zwischen den beiden Teilelektroden 4, 6 bei an den Körper des Patienten angelegten Teilelektroden 4; 6 durchgeführt. Ferner kann auch eine Bestimmung einer direkten Übergangsimpedanz mit aneinander anliegenden Teilelektroden 4, 6 bestimmt werden, wobei die Anlageflächen der beiden Teilelektroden 4, 6 aneinander anliegen und die elektrische Verbindung zwischen diesen beispielsweise durch eine dazwischenliegende Gelschicht hergestellt wird.

Ferner ist eine Neutralelektrodenzuleitung 10 vorgesehen, die sich an einer Verzweigung 12 in zwei Zweige 14, 16 aufteilt. Die Zweige 14, 16 führen jeweils zu einer Teilelektrode 4, 6, so dass elektrische Ladung von den Teilelektroden 4, 6 über die Zweige 14, 16 und die Neutralelektrodenzuleitung 10 an den geerdeten Teil eines Hochfrequenz-Chirurgiegerätes, insbesondere einer Hochfrequenz-Stromversorgung, rückführbar ist. In jedem Zweig 14, 16 der Neutralelektrodenzuleitung 10 ist je ein Koppelkondensator 18, 20 angeordnet.

Die Teilelektroden 4, 6, die Zweige 14, 16, die Koppelkondensatoren 18, 20 und die Neutralelektrodenzuleitung 10 bilden also einen Teil eines Hochfrequenz-Stromkreises. Bei Hochfrequenz-Anwendungen werden diese von hochfrequenten Wechselströmen durchflossen.

Die Koppelkondensatoren werden durch eine dazu parallel geschaltete Messspule 22 zu einem Basisschwingkreis 24 der Messvorrichtung 2 ergänzt. Parallel zu dem Basisschwingkreis 24 sind die beiden Teilelektroden 4, 6 parallel zuschaltbar, so dass der Basisschwingkreis 24 durch eine zwischen den beiden Teilelektroden 4, 6 auftretende Übergangsimpedanz zu einem erweiterten Schwingkreis 25 erweiterbar ist.

Von einer Stromversorgungseinrichtung 28 ist über eine erste Zuleitung 26 eine Mess-Wechselspannung direkt in den Basisschwingkreis 24 bzw. in den erweiterten Schwingkreis 25 einprägbar. Ein Kondensator 30 in der ersten Zuleitung 26 dient als Gleichstromsperre. Eine zweite Zuleitung 32 führt von dem Basisschwingkreis 24 bzw. dem erweiterten Schwingkreis 25 zu Masse, so dass der Basisschwingkreis 24 bzw. der erweiterte Schwingkreis 25 zwischen der ersten Zuleitung 26 und der zweiten Zuleitung 32 in Reihe geschaltet ist.

Die Messvorrichtung 2 weist ferner eine Steuerung in Form eines Mikrokontrollers 34 auf, der zur Frequenzsteuerung der Stromversorgungseinrichtung 28, zum Feststellen des Strom- und Spannungsnulldurchgangs in der ersten Zuleitung 26, zur Erfassung des Spannungswertes in der Zuleitung 26 und zur Erfassung der von den jeweiligen Teilelektroden 4, 6 abfließenden Teilströme ausgebildet ist.

Die Stromversorgungseinrichtung 28 ist eine spannungsgesteuerte Konstantstromquelle, die ein rechteckförmiges Stromsignal ausgibt und deren Frequenz durch ein Timersignal einstellbar ist. Die Stromversorgungseinrichtung 28 ist vorzugsweise ausgelegt, Spannungswerte von maximal $\pm 10V$ auszugeben. Zur Frequenzsteuerung ist in dem Mikrokontroller 34 ein Timer 36 vorgesehen, der ein Timersignal an die Stromversorgungseinrichtung 28 zur Frequenzsteuerung der Mess-Wechselspannung aussendet. Ferner weist die Messvorrichtung 2 eine von dem Mikrokontroller 34 angesteuerte Pegelanpassung 38 auf, welche die Einstellung verschiedener Messströme und damit verschiedener Messbereiche ermöglicht. Dadurch kann auch die hohe Übergangsimpedanz einer nicht applizierten Neutralelektrode 8 erfasst werden, bei der die elektrische Verbindung zwischen den beiden Teilelektroden 4, 6 beispielsweise durch eine Gelbrücke zwischen den beiden Anlageflächen der Teilelektroden 4, 6 hergestellt wird.

Der Mikrokontroller 34 erfasst über einen Analogkomparator 40 den Spannungsnulldurchgang in einer Zuleitung 26 zum Basisschwingkreis 24 bzw. zum erweiterten Schwingkreis 25. Demgemäß liegen dem Mikrokontroller 34 die Phaseninformationen zwischen Strom und Spannung vor. Der Mikrokontroller 34 ist derart ausgelegt, dass er gemäß einem Proportional-Integral-Regelalgorithmus die Ausgangsfrequenz der Stromversorgungseinrichtung 28 über das Timersignal derart verändert, dass der Phasenunterschied zwischen Strom und Spannung zu Null wird. Bei Phasengleichzeit zwischen Strom und Spannung wird der Basisschwingkreis 24 bzw. der erweiterte Schwingkreis 25 in seiner jeweiligen Resonanzfrequenz, d.h. in seiner Basisresonanzfrequenz bzw. in seiner Probenresonanzfrequenz betrieben.

Die Messvorrichtung 2 wird auf Patientenkreispotential betrieben. Gegenüber einem Zwischenstromkreis des Hochfrequenz-Chirurgiegerätes sind die Stromversorgungseinrichtung 28 und der Mikrokontroller 34 jedoch galvanisch getrennt. Dazu dienen ein DC/DC-Wandler 42 und Optokoppler 44.

Mit den bisher beschriebenen Schaltungsteilen der Messvorrichtung 2 ist eine erfindungsgemäße Messung des kapazitiven Anteils der Übergangsimpedanz zwischen den beiden Teilelektroden 4, 6 durchführbar.

Bei nicht applizierten Teilelektroden 4, 6 ist die auftretende Impedanz zwischen den Teilelektroden 4, 6 derart groß, dass der Basisschwingkreis 24 durch die Teilelektroden 4, 6 nicht beeinflusst wird. Demgemäß ist bei nicht applizierten Teilelektroden 4, 6 die Basisresonanzfrequenz des Basisschwingkreises 24, der aus der Kopplungskapazität $C_0$ der Koppelkondensatoren 18, 20 und der Messinduktivität L der Messspule 22 gebildet wird, bestimmbar.

Hierzu erfasst der Mikrokontroller 34 den Phasenunterschied zwischen Strom und Spannung der an den Basisschwingkreis 24 angelegten Mess-Wechselspannung und verändert gemäß dem Proportional-Integral-Regelalgorithmus die Ausgangsfrequenz der Stromversorgungseinrichtung 28 derart, dass der Phasenunterschied zwischen Strom und Spannung Null wird. Anhand des Timersignals ist demnach die Basisresonanzfrequenz $f_1$ des Basisschwingkreises 24 bekannt.

Bei applizierten Teilelektroden 4, 6 tritt zwischen diesen die Übergangsimpedanz auf, deren Bestimmung durch die Messvorrichtung 2 erfolgt.

Hierzu wird an den erweiterten Schwingkreis 25, der aus der Übergangsimpedanz zwischen den Teilelektroden 4, 6, der Kopplungskapazität $C_0$ der Koppelkondensatoren 18, 20 und der Messinduktivität L der Messspule 22 gebildet wird, eine Mess-Wechselspannung angelegt. Der Mikrokontroller 34 erfasst den Phasenunterschied zwischen Strom und Spannung und verändert wiederum gemäß dem Proportional-Integral-Regelalgorithmus die Ausgangsfrequenz der Stromversorgungseinrichtung 28 derart, dass der Phasenunterschied zwischen Strom und Spannung Null wird. Anhand des Timersignals ist die Probenresonanzfrequenz des erweiterten Schwingkreises 25 bekannt.

Die Kapazität $C_x$ des Übergangs zwischen den beiden Teilelektroden 4, 6 wird anhand nachfolgender Formel berechnet, wobei die Basisresonanzfrequenz mit $f_1$ und die Probenresonanzfrequenz mit $f_2$ bezeichnet wird:

$$C_x = \frac{1}{4\pi^2 L}\left(\frac{f_1^{\,2} - f_2^{\,2}}{f_1^{\,2} f_2^{\,2}}\right)$$

Der kapazitive Widerstand des Übergangs für eine bestimmte Frequenz f wird anhand folgender Formel ermittelt:

$$X_C = \frac{1}{2\pi f C_x}$$

Der gezeigten Berechnung liegt die Annahme zugrunde, dass die Übergangsimpedanz durch eine Parallelschaltung aus einem ohmschen Widerstand $R_x$ und einer Kapazität $C_x$ beschreibbar ist.

Für die Bestimmung des ohmschen Anteils der Übergangsimpedanz weist die Messvorrichtung 2 einen Schaltungsteil 46 zum Messen der Spannung über dem Basisschwingkreis 24 bzw. dem erweiterten Schwingkreis 25 auf. Der Schaltungsteil 46 weist eine Spitzenspannungsbegrenzung 48 zur Unterdrückung von Hochfrequenzstörungen und eine Spitzenwerterfassung auf, die aus einem Synchrongleichrichter 50 und einem Tiefpass 52 gebildet wird. Der Synchrongleichrichter 50 wird vom Mikrokontroller 34 durch ein von dem Timersignal abgeleitetes Signal angesteuert. Der Mikrokontroller 34 weist ferner einen Analog/Digital-Wandler 54 und einen UART (universal asynchronous receivertransmitter: universeller asynchroner Empfänger-Sender) 56 auf, wobei das Messsignal des Schaltungsteils 46 durch den A/D-Wandler 54 digitalisiert wird und mit Hilfe des UART 56 an eine (nicht gezeigte) Steuereinrichtung des Hochfrequenz-Chirurgiegeräts übermittelt wird.

Der ohmsche Widerstand $R_x$ der Übergangsimpedanz zwischen den beiden Elektroden wird aus nachfolgender Formel erhalten:

$$R_X = \frac{U_s}{I_s}$$

$U_s$ ist der Spitzenwert der Wechselspannung über dem Schwingkreis und $I_s$ der Spitzenwert der Stromstärke. Der Spitzenwert der Wechselspannung ist über das Schaltungsteil 46 messbar, während der Spitzenwert der Stromstärke $I_s$ aus der Einstellung der Stromversorgungseinrichtung 28, die als Konstantstromquelle ausgebildet ist, bekannt ist.

Ferner ist die gezeigte Messvorrichtung 2 ausgebildet, jeweils den von einer Teilelektrode abfließenden hochfrequenten Strom zu messen. Zur Erfassung der Teilströme, insbesondere während einer Hochfrequenz-Anwendung, dienen die Stromsensoren 58, 60, die in den Zweigen 14, 16 der Neutralelektrodenzuleitung 10 angeordnet sind. Das Ausgangssignal der Stromsensoren 58, 60 wird jeweils einer zugehörigen Spitzenwert-Erfassungsschaltung 62, 64 zugeführt, wobei ein Ausgangssignal der Spitzenwert-Erfassungsschaltungen 62, 64 wiederum an den A/D-Wandler 54 gesendet wird. Demgemäß sind die Teilströme, die über die einzelnen Teilelektroden 4, 6 abfließen, von dem Mikrokontroller 34 erfassbar. Ferner werden die von dem A/D-Wandler 54 digitalisierten Messwerte bezüglich der Teilströme durch den UART 56 seriell der (nicht gezeigten) Steuereinrichtung des Hochfrequenz-Chirurgiegerätes zur Auswertung zur Verfügung gestellt.

Durch die vorgesehene Überwachung der Symmetrie der hochfrequenten Teilströme, die während einer Hochfrequenz-Applikation in den beiden Zweigen 14, 16 der Neutralelektrodenzuleitung 10 auftreten, kann ein Ablösen einer Teilelektrode 4, 6 erkannt werden. Die Symmetrie kann durch Verhältnisbildung der beiden Teilströme beurteilt werden. Durch Summation der Teilströme ist der gesamte HF-Strom, der durch die neutrale Elektrode fließt, berechenbar. Der Gesamtstrom kann bei einer monopolaren Hochfrequenz-Anwendung zur Plausibilitätsprüfung eines Messwertes für den Ausgangsstrom der Aktivelektrode dienen und durch Vergleich mit diesem einen Rückschluss auf die Höhe eines Leckstromes ermöglichen.

Die Erfindung ist nicht auf die beispielhaft gezeigte Ausführungsform der Figur 1 beschränkt. Die Erfindung ergibt sich vielmehr aus einer fachmännischen Gesamtbetrachtung der Ansprüche, der Beschreibung, der beispielhaften Ausführungsform, und der nachfolgend erwähnten Varianten, die einem Fachmann Hinweise auf weitere alternative Ausführungsformen geben sollen.

Insbesondere ist die angegebene Reihenfolge, nach der die Basisresonanzfrequenz zeitlich vor der Probenresonanzfrequenz gemessen wird, nicht zwingend. Vielmehr kann auch zuerst die Probenresonanzfrequenz und nachfolgend die Basisresonanzfrequenz gemessen werden. Hierzu ist vorteilhaft, wenn ein paralleles Zuschalten der Teilelektroden durch einen Schalter erfolgt, so dass die Teilelektroden nach dem Messen der Probenresonanzfrequenz durch Öffnen des Schalters von dem Basisschwingkreis trennbar sind und zum Messen der Basisresonanzfrequenz ein Abnehmen der Teilelektroden von dem Gewebe nicht notwendig ist. Vorzugsweise wird nach Beendigung der Hochfrequenz-Anwendung eine nochmalige Kontrollmessung der Basisresonanzfrequenz durchgeführt.

Ferner kann als Ausgangssignal der Stromversorgungseinrichtung auch ein sinusförmiges Stromsignal verwendet werden.

Die für den Mikrokontroller beschriebenen Funktionen können auch ganz oder teilweise von der Steuereinrichtung des Hochfrequenz-Chirurgiegerätes durchgeführt werden. Insbesondere kann vorgesehen sein, dass der Mikrokontroller der Messvorrichtung in die Steuereinrichtung des Hochfrequenz-Chirurgiegerätes integriert ist.

Bezugszeichenliste

| | |
|---|---|
| 2 | Messvorrichtung |
| 4, 6 | Teilelektroden |
| 8 | Neutralelektrode |
| 10 | Neutralelektrodenzuleitung |
| 12 | Verzweigung |
| 14, 16 | Zweige |
| 18, 20 | Koppelkondensatoren |
| 22 | Messspule |
| 24 | Basisschwingkreis |
| 25 | erweiterter Schwingkreis |
| 26 | erste Zuleitung |
| 28 | Stromversorgungseinrichtung |
| 30 | Kondensator |
| 32 | zweite Zuleitung |
| 34 | Mikrokontroller |
| 36 | Timer |
| 38 | Pegelanpassung |
| 40 | Analogkomparator |
| 42 | DC/DC-Wandler |
| 44 | Optokoppler |
| 46 | Schaltungsteil |
| 48 | Spitzenspannungsbegrenzung |
| 50 | Synchrongleichrichter |
| 52 | Tiefpass |
| 54 | A/D-Wandler |

56      UART
58, 60   Stromsensoren
62, 64   Spitzenwert-Erfassungsschaltung


**Patentansprüche**

1.  Verfahren zur Bestimmung der Übergangsimpedanz zwischen zwei Teilelektroden (4, 6) einer geteilten Neutralelektrode (8) in der Hochfrequenz-Chirurgie,
    **gekennzeichnet durch**
    ein Bestimmen des ohmschen Anteils und des rein kapazitiven Anteils der Übergangsimpedanz, wobei hierzu in einem Schwingkreis (24, 25) eine Resonanzfrequenz-Verschiebung gemessen wird, die durch ein paralleles Zuschalten der beiden Teilelektroden in den Schwingkreis bedingt ist.

2.  Verfahren nach Anspruch 1,
    **dadurch gekennzeichnet, dass**
    das Bestimmen des rein kapazitiven Anteils der Übergangsimpedanz folgende Schritte aufweist:

    Bereitstellen eines Basisschwingkreises (24) mit einer Messinduktivität (22) und einer Kopplungskapazität (18, 20);
    Messen einer Basisresonanzfrequenz des Basisschwingkreises;
    Bereitstellen eines erweiterten Schwingkreises (25) durch paralleles Zuschalten der Übergangsimpedanz in den Basisschwingkreis;
    Messen einer Probenresonanzfrequenz des erweiterten Schwingkreises;
    Ermitteln des kapazitiven Anteils aus der Basisresonanzfrequenz, der Probenresonanzfrequenz und der Messinduktivität unter der Annahme, dass die Übergangsimpedanz aus einem ohmschen Widerstand und einem kapazitiven Widerstand gebildet wird, die zueinander parallel geschaltet sind.

3.  Verfahren nach Anspruch 1 oder 2,
    **dadurch gekennzeichnet, dass**
    zum Messen der Basisresonanzfrequenz des Basisschwingkreises (24) bzw. der Probenresonanzfrequenz des erweiterten Schwingkreises (25) Mess-Wechselspannung an den Basisschwingkreis bzw. an den erweiterten Schwingkreis angelegt wird, die Phasenlage zwischen Strom und Spannung in einer Zuleitung (26, 32) zu dem jeweiligen Schwingkreis gemessen wird und die Frequenz der Mess-Wechselspannung angepasst wird, bis Strom und Spannung phasengleich sind.

4.  Verfahren nach Anspruch 3,
    **dadurch gekennzeichnet, dass**
    zum Messen der Basisresonanzfrequenz des Basisschwingkreises (24) bzw. der Probenresonanzfrequenz des erweiterten Schwingkreises (25) ein rechteckförmiges Wechselstromsignal konstanter Stromamplitude an den Basisschwingkreis bzw. an den erweiterten Schwingkreis angelegt wird.

5.  Verfahren nach Anspruch 4,
    **dadurch gekennzeichnet, dass**
    die Frequenz des Wechselstromsignals mittels eines Timersignals einstellbar ist, so dass der Nulldurchgang und die Frequenz des Wechselstromsignals durch das Timersignal bekannt sind.

6.  Verfahren nach Anspruch 1 oder 2,
    **dadurch gekennzeichnet, dass**
    zum Messen der Basisresonanzfrequenz des Basisschwingkreises (24) bzw. der Probenresonanzfrequenz des erweiterten Schwingkreises (25) eine Mess-Wechselspannung an den Basisschwingkreis bzw. an den erweiterten Schwingkreis angelegt wird, die Spannung über dem jeweiligen Schwingkreis gemessen wird und die Frequenz der Mess-Wechselspannung angepasst wird, bis die Spannung einen Maximalwert erreicht.

7.  Verfahren nach einem der vorangehenden Ansprüche,
    **dadurch gekennzeichnet, dass**
    der ohmsche Anteil der Übergangsimpedanz bestimmt wird, wobei hierzu bei der Probenresonanzfrequenz die Spannung über dem erweiterten Schwingkreis und der Strom in einer Zuleitung (26, 32) zu dem erweiterten Schwingkreis (25) gemessen werden und aus diesen der ohmsche Anteil ermittelt wird.

8.  Verfahren nach einem der Ansprüche 2 bis 7,
    **dadurch gekennzeichnet, dass**
    zur Überwachung der Übergangsimpedanz in zeitlichen Abständen die Probenresonanzfrequenz des erweiterten Schwingkreises (25) gemessen wird.

9.  Verfahren nach einem der vorangehenden Ansprüche,
    **dadurch gekennzeichnet, dass**
    während einer Hochfrequenz-Chirurgieanwendung jeweils der von einer Teilelektrode (4, 6) abfließende Strom gemessen wird.

10. Messvorrichtung mit einer zwei Teilelektroden (4, 6) umfassenden Neutralelektrode (8) für eine Hochfrequenz-Chirurgieanwendung, wobei die Messvorrichtung zur Bestimmung der Übergangsimpedanz zwischen den zwei Teilelektroden (4, 6) der geteilten Neutralelektrode (8) ausgelegt ist, mit einem Schwingkreis (24), in welchen die beiden Teilelektroden parallel zuschaltbar sind, und mit einer Stromversorgungseinrichtung (28), mittels welcher eine Mess-Wechselspannung in den Schwingkreis (24, 25) einprägbar ist;

**dadurch gekennzeichnet, dass**

die Messvorrichtung zum Bestimmen des ohmschen Anteils und des rein kapazitiven Anteils der Übergangsimpedanz ausgebildet ist, wobei sie eine Steuerung (34) und eine Phasenmesseinrichtung (36, 40) aufweist, so dass die Phase des Stromes und der Spannung von der Steuerung über die Phasenmesseinrichtung erfassbar ist und die Frequenz der Stromversorgungseinrichtung durch die Steuerung derart steuerbar ist, dass Strom und Spannung in Phase sind.

11. Messvorrichtung nach Anspruch 10,

**dadurch gekennzeichnet, dass**

die Messvorrichtung derart ausgebildet ist, dass die Mess-Wechselspannung von der Stromversorgungseinrichtung (28) direkt in den Schwingkreis (24; 25) eingespeist wird.

12. Messvorrichtung nach Anspruch 10 oder 11,

**dadurch gekennzeichnet, dass**

der Schwingkreis eine Kopplungskapazität und eine dazu parallel geschaltete Messinduktivität (22) aufweist, wobei die Kopplungskapazität durch zwei Koppelkondensatoren (18, 20) gebildet wird, die jeweils in einem Zweig einer verzweigten Neutralelektrodenzuleitung (10) angeordnet sind.

13. Messvorrichtung nach einem der Ansprüche 10 bis 12,

**dadurch gekennzeichnet, dass**

die Stromversorgungseinrichtung (28) eine Konstantstromquelle ist, deren Frequenz mittels eines Timersignals von der Steuerung (34) einstellbar ist und die als Ausgang einen rechteckförmigen Wechselstrom erzeugt, und dass ein Analogkomparator (40) zum Erfassen des Spannungsnulldurchgangs vorgesehen ist, so dass durch die Steuerung mittels des Timersignals und des Analogkomparators der Phasenunterschied zwischen Strom und Spannung erfassbar ist.

14. Messvorrichtung nach einem der Ansprüche 10 bis 13,

**dadurch gekennzeichnet, dass**

eine Spannungsmesseinrichtung (46) zum Messen der Spannung über dem Schwingkreis (24; 25) vorgesehen ist, so dass ein Spitzenwert der Spannung bei Resonanzbedingung des Schwingkreises mit zugeschalteter Übergangsimpedanz messbar ist.

15. Messvorrichtung nach einem der Ansprüche 10 bis 14,

**dadurch gekennzeichnet, dass**

an jedem Leitungszweig (14, 16), der zu einer Teilelektrode (4, 6) der Neutralelektrode (8) führt, ein Stromsensor (58, 60) angeordnet ist, mittels welchem der von der jeweiligen Teilelektrode abfließende Strom messbar ist.

16. Hochfrequenz-Chirurgiegerät mit mindestens einer Aktivelektrode und einer geteilten Neutralelektrode (8),

**gekennzeichnet durch**

eine Messvorrichtung (2) zur Bestimmung der Übergangsimpedanz zwischen zwei Teilelektroden (4, 6) der geteilten Neutralelektrode (8) nach einem der Ansprüche 10 bis 15.

**Claims**

1. Method of determining the transient impedance between two partial electrodes (4, 6) of a divided neutral electrode (8) in high-frequency surgery,

**characterised by**

determination of the ohmic component and the purely capacitive component of the transient impedance, wherein to this end a resonant frequency displacement, which is caused by a parallel connection of the two electrodes in an oscillating circuit (24, 25), is measured by the oscillating circuit.

2. Method according to Claim 1,

**characterised in that**

the determination of the purely capacitive component of the transient impedance comprises the following steps:

provision of a basic oscillating circuit (24) with a measurement inductance (22) and a coupling capacitance (18, 20); measurement of a basic resonant frequency of the basic oscillating circuit; provision of an expanded oscillating circuit (25) by parallel connection of the transient impedance to the basic oscillating circuit; measurement of a sample resonant frequency of the expanded oscillating circuit; calculation of the capacitive component of the basic resonant frequency, the sample resonant frequency and the measurement inductance, on the assumption that the transient impedance is formed from an ohmic resistance and a capacitive resistance that are connected in parallel.

3. Method according to Claim 1 or 2,

**characterised in that,**

to measure the basic resonant frequency of the basic oscillating circuit (24) or the sample resonant frequency of the expanded oscillating circuit (25), measurement alternating voltage is applied to the basic oscillating circuit or the expanded oscillating circuit, the phase relation between current and voltage is measured in a lead (26, 32) to the respective oscillating circuit, and the frequency of the measurement alternating voltage is adjusted until current and voltage are in phase.

4. Method according to Claim 3,
**characterised in that,**
to measure the basic resonant frequency of the basic oscillating circuit (24) or the sample resonant frequency of the expanded oscillating circuit (25), a rectangular alternating current signal with a constant current amplitude is applied to the basic oscillating circuit or to the expanded oscillating circuit.

5. Method according to Claim 4,
**characterised in that**
the frequency of the alternating current signal can be adjusted by means of a timer signal, so that the zero crossing and the frequency of the alternating current signal are known from the timer signal.

6. Method according to Claim 1 or 2,
**characterised in that,**
to measure the basic resonant frequency of the basic oscillating circuit (24) or the sample resonant frequency of the expanded oscillating circuit (25), a measurement alternating voltage is applied to the basic oscillating circuit or the expanded oscillating circuit, the voltage is measured across the respective oscillating circuit and the frequency of the measurement alternating voltage is adjusted until the voltage reaches a maximum value.

7. Method according to one of the previous claims,
**characterised in that**
the ohmic component of the transient impedance is determined, wherein to this end the voltage across the expanded oscillating circuit and the current in a lead (26, 32) to the expanded oscillating circuit (25) are measured for the sample resonant frequency, and from these the ohmic component is calculated.

8. Method according to one of the Claims 2 to 7,
**characterised in that,**
to monitor the transient impedance at temporal intervals, the sample resonant frequency of the expanded oscillating circuit (25) is measured.

9. Method according to one of the previous claims
**characterised in that,**
during a high-frequency surgical procedure, the current flowing away from a partial electrode (4, 6) is measured each time.

10. Measuring device with a neutral electrode (8) comprising two partial electrodes (4, 6), wherein the measuring device for determining the transient impedance between the two partial electrodes (4, 6) of the divided neutral electrode (8) is configured with an oscillating circuit (24), in which the two partial electrodes are connected in parallel, and with a current supply unit (28), by means of which a measurement alternating voltage can be impressed into the oscillating circuit (24, 25);
**characterised in that**
the measuring device is configured for determining the ohmic component and the purely capacitive component of the transient impedance, wherein it has a control system (34) and a phase measuring unit (36, 40), so that the phase of the current and of the voltage can be detected by the control system by means of the phase measuring unit and the frequency of the current supply unit can be controlled by the control system in such a way that current and voltage are in phase.

11. Measuring device according to Claim 10,
**characterised in that**
the measuring device is configured in such a way that the measurement alternating voltage is fed directly into the oscillating circuit (24; 25) by the current supply unit (28).

12. Measuring device according to Claim 10 or 11,
**characterised in that**
the oscillating circuit has a coupling capacitance and a measurement inductance (22) connected in parallel, wherein the coupling capacitance is formed by two coupling condensers (18, 20), each of which is arranged in a branch of a divided neutral electrode lead (10).

13. Measuring device according to one of the Claims 10 to 12,
**characterised in that**
the current supply unit (28) is a constant current source, the frequency of which can be adjusted by the control system (34) by means of a timer signal and which generates a rectangular alternating current as an output, and **in that** an analog comparator (40) is provided for detecting the voltage zero crossing, so that the phase difference between current and voltage can be detected by the control system by means of the timer signal and the analog comparator.

14. Measuring device according to one of the Claims 10 to 13,
**characterised in that**
a voltage measuring unit (46) is provided for measuring the voltage across the oscillating circuit (24, 25), so that a peak value of the voltage can be measured for the resonant condition of the oscillating circuit with transient impedance incorporated.

15. Measuring device according to one of the Claims 10 to 14,
**characterised in that**
at each branch of the lead (14, 16) leading to a partial electrode (4, 6) of the neutral electrode (8), a current sensor (58, 60) is arranged, by means of which the current flowing away from the respective partial electrode can be measured.

16. High-frequency surgery appliance with at least one active electrode and a divided neutral electrode (8),
**characterised by**
a measuring device (2) for determining the transient impedance between two partial electrodes (4, 6) of the divided neutral electrode (8) according to one of the Claims 10 to 15.

**Revendications**

1. Procédé de détermination de l'impédance transitoire entre deux électrodes partielles (4, 6) d'une électrode neutre divisée (8) en chirurgie à haute fréquence,
**caractérisé par**
une détermination de la partie ohmique et de la partie purement capacitive de l'impédance transitoire, moyennant quoi, dans un circuit oscillant (24, 25), un décalage de fréquence de résonance est mesuré, lequel est conditionné à une mise sous tension en parallèle des deux électrodes partielles dans le circuit oscillant.

2. Procédé selon la revendication 1,
**caractérisé en ce que**
la détermination de la partie purement capacitive de l'impédance transitoire comprend les étapes consistant à :

préparer un circuit oscillant de base (24) ayant une inductance de mesure (22) et une capacité de couplage (18, 20) ;
mesurer une fréquence de résonance de base du circuit oscillant de base ;
préparer un circuit oscillant agrandi (25) par mise sous tension en parallèle de l'impédance transitoire dans le circuit oscillant de base ;
mesurer une fréquence de résonance d'essai du circuit oscillant agrandi ;
déterminer la partie capacitive à partir de la fréquence de résonance de base, de la fréquence de résonance d'essai et de l'inductance de mesure en supposant que l'impédance transitoire est formée d'une résistance ohmique et d'une résistance capacitive montées en parallèle.

3. Procédé selon la revendication 1 ou 2,
**caractérisé en ce que,**
pour mesurer la fréquence de résonance de base du circuit oscillant de base (24) respectivement la fréquence de résonance d'essai du circuit oscillant agrandi (25), on applique une tension alternative de mesure au circuit oscillant de base respectivement au circuit oscillant agrandi, on mesure la position de phase entre le courant et la tension dans un câble d'alimentation (26, 32) du circuit oscillant et on ajuste la fréquence de la tension alternative de mesure jusqu'à ce que le courant et la tension soit en phase.

4. Procédé selon la revendication 3,
**caractérisé en ce que,**
pour mesurer la fréquence de résonance de base du circuit oscillant (24) respectivement la fréquence de résonance d'essai du circuit oscillant agrandi (25), on applique un signal alternatif carré ayant une amplitude de courant constante au circuit oscillant de base respectivement au circuit oscillant agrandi.

5. Procédé selon la revendication 4,
**caractérisé en ce que,**
la fréquence du signal de courant alternatif peut être réglée au moyen d'un signal de minuterie, de sorte que le passage au point zéro et la fréquence du signal de courant alternatif sont connus grâce au signal de minuterie.

6. Procédé selon la revendication 1 ou 2,
**caractérisé en ce que,**
pour mesurer la fréquence de résonance de base du circuit oscillant de base (24) respectivement la fréquence de résonance d'essai du circuit oscillant agrandi (25), on applique une tension alternative de mesure au circuit oscillant de base respectivement au circuit oscillant agrandi, on mesure la tension aux bornes du circuit oscillant et on ajuste la fréquence de la tension alternative de mesure jusqu'à ce que la tension atteigne une valeur maximale.

7. Procédé selon l'une quelconque des revendications précédentes,
**caractérisé en ce que,**
on détermine la partie ohmique de l'impédance transitoire, moyennant quoi, pour la fréquence de résonance d'essai, on mesure la tension aux bornes du circuit oscillant agrandi et le courant dans un câble d'alimentation (26, 32) du circuit oscillant agrandi (25) et on détermine à partir de cela la partie ohmique.

8. Procédé selon l'une quelconque des revendications 2 à 7,
**caractérisé en ce que,**
pour surveiller l'impédance transitoire dans des intervalles de temps, on mesure la fréquence de résonance d'essai du circuit oscillant agrandi (25).

9. Procédé selon l'une quelconque des revendications précédentes,

**caractérisé en ce que,**
on mesure le courant sortant d'une électrode partielle (4, 6) pendant une application en chirurgie à haute fréquence.

**10.** Dispositif de mesure doté d'une électrode neutre (8) comprenant deux électrodes partielles (4, 6) pour une application en chirurgie à haute fréquence, dans lequel
le dispositif de mesure pour déterminer l'impédance transitoire entre les deux électrodes partielles (4, 6) de l'électrode neutre divisée (8) comprend un circuit oscillant (24), dans lequel les deux électrodes partielles peuvent être mises sous tension en parallèle et un dispositif d'alimentation en courant électrique (28) à l'aide duquel on peut appliquer une tension alternative de mesure dans le circuit oscillant (24, 25) ;
**caractérisé en ce que,**
le dispositif de mesure pour déterminer la partie ohmique et la partie purement capacitive de l'impédance transitoire est constitué de manière à présenter une commande (34) et un dispositif de mesure de phase (36, 40), de sorte que la phase du courant et de la tension provenant de la commande aux bornes du dispositif de mesure de phase peut être enregistrée et que la fréquence du dispositif d'alimentation en courant électrique peut être commandée par la commande, afin que la tension et le courant soient en phase.

**11.** Dispositif de mesure selon la revendication 10,
**caractérisé en ce que,**
le dispositif de mesure est constitué de sorte que la tension alternative de mesure provenant du dispositif d'alimentation en courant électrique (28) est directement alimentée dans le circuit oscillant (24 ; 25).

**12.** Dispositif de mesure selon la revendication 10 ou 11,
**caractérisé en ce que,**
le circuit oscillant comprend une capacité de couplage et une inductance de mesure (22) montée en parallèle, moyennant quoi la capacité de couplage est formée par deux condensateurs (18, 20) agencés dans une branche d'un câble électrique d'électrode neutre (10).

**13.** Dispositif de mesure selon l'une quelconque des revendications 10 à 12,
**caractérisé en ce que,**
le dispositif d'alimentation en courant électrique (28) est une source de courant constant dont la fréquence peut être réglée au moyen d'un signal de minuterie provenant de la commande (34) et qui la produit en tant que sortie d'un courant alternatif carré et **en ce que** le comparateur analogique (40) est prévu pour déterminer le passage au point zéro de la tension, de sorte que la différence de phase entre le courant et la tension peut être enregistrée grâce à la commande au moyen du signal de minuterie et du comparateur analogique.

**14.** Dispositif de mesure selon l'une quelconque des revendications 10 à 13,
**caractérisé en ce que,**
un dispositif de mesure de tension (46) pour mesurer la tension aux bornes du circuit oscillant (24 ; 25) est prévu, de sorte qu'une valeur de crête de la tension dans une condition de résonance du circuit oscillant peut être mesurée avec l'impédance transitoire mise sous tension.

**15.** Dispositif de mesure selon l'une quelconque des revendications 10 à 14,
**caractérisé en ce que,**
à chaque branche de ligne (14, 16) conduisant à une électrode partielle (4, 6) de l'électrode neutre (8), un capteur de courant (58, 60) est agencé, au moyen duquel on peut mesurer le courant sortant de l'électrode partielle.

**16.** Appareil pour chirurgie à haute fréquence comportant au moins une électrode active et une électrode neutre divisée (8),
**caractérisé par**
un dispositif de mesure (2) pour déterminer l'impédance transitoire entre deux électrodes partielles (4, 6) de l'électrode neutre divisée (8) selon l'une quelconque des revendications 10 à 15.

Fig. 1

**IN DER BESCHREIBUNG AUFGEFÜHRTE DOKUMENTE**

**In der Beschreibung aufgeführte Patentdokumente**

- DE AS1139927 B
- US 3913583 A
- US 3933157 A
- US 5087257 A
- WO 9619152 A
- US 4200104 A
- DE 19714972 A1
- WO 2004028385 A1